# EUROPEAN PATENT APPLICATION

(11) **EP 4 604 116 A1**
(43) Date of publication of application: **20.08.2025**
(21) Application number: 24219145.0
(22) Date of filing: 11.12.2024
(51) Int. Cl.: G10H 1/00, A61B 5/11

(54) **APPARATUS FOR GENERATING AI-GENERATED MUSIC BASED ON GAIT**

(30) Priority: 17.02.2024 TW 113105631
(71) Applicant: Decentralized Biotechnology Intelligence Co., Ltd., Taipei 106021 (TW)
(72) Inventor: CHOU, Yao Sheng, 106021 Taipei (TW); WU, Chung Yuan, 106021 Taipei (TW); CHOU, Yen Han, 106021 Taipei (TW); JIANG, Lin Yi, 106021 Taipei (TW); CHOI, Kang, 106021 Taipei (TW)
(74) Representative: Metida

(57) **Abstract**

The present invention discloses an apparatus for producing AI-generated music based on gait, which includes a sensor assembly disposed on the user's foot, a pressure sensing device and an inertial sensing module used to collect the user's movement related data, a computing electronic device is coupled to the sensor assembly for receiving the user's movement-related data and the gait analysis module is used to analyze the movement-related data to obtain the user's gait parameters, and then extracting the rhythm parameters from the user's gait parameters by a rhythm parameter extraction module, and an AI music generation module is coupled to the computing electronic device for receiving the user's motion-related rhythm parameters, thereby converting the motion-related rhythm parameters into corresponding number of beats, high and low notes, light and heavy notes. A piece of music is created by an AI algorithm.

## Description

### TECHNICAL FIELD

The present invention relates to an AI-generated music, more specifically, an apparatus to create an AI-generated music based on gait.

### BACKGROUND

The information related to the people's movement gait can be detected and extracted through various electronic sensors, information and communication technologies. Previous technologies, such as US patent US 5765300, have attempted to develop electronic tap dance shoes by pressure sensors to detect dancers' activities, and thereby sending corresponding MIDI (Musical Instrument Digital Interface) notes by wired or wireless means. For example, the patent provides a shoe-activated sound synthesizer device that converts shoe movement into audible sounds. A shoe includes at least one trigger disposed and capable of generating a trigger signal when the shoe is bent to a predetermined degree. The shoe flexes when it contacts with the floor. By contacting the floor via different parts of the shoe, the music is generated by the trigger elements contained within the different positions of the shoe under control manner. A sound synthesizer circuit is coupled to each trigger elements contained within the shoe. When a trigger signal is received from the shoe, the sound synthesizer circuit produces an audible sound through the speaker.

The pressure sensors may also be embedded in the floor or on surfaces of the floor to act as home game controllers or as tracking devices to track the user's movements and activities for fitness training purpose. The pressure sensors may transmit data packets to the receivers of a mobile device wirelessly.

Music and dance are enjoyable activities in modern society. Learning to dance is one of the most popular activities in the world. If using your favorite music to dance, the learning effect is particularly good, however, some dance moves are not easy to learn.

It is a complex process for achieving harmonious movements when a user's body moves during dancing or exercising, the dancer has to consider the coordination of action with time.

The connection between rhythm and people's daily life is getting closer and closer. For example, the people may exercise with the rhythm of the audio, the video games can be designed based on the rhythm of the audio, and the lights are set to flash with the rhythm of the audio, etc. In order to fetch the rhythm of audio, it is necessary to determine the rhythm point from the audio, typically. Therefore, what is required is to accurately determine the rhythm point of the audio in the related technical fields.

It is not easy to automatically generate a piece of music. In addition to melody generation, other elements such as multi-track accompaniment generation, lyric generation, orchestration and arrangement are also necessary to be considered. However, due to the rapid development of artificial intelligence technology, it has become possible to employ generative artificial intelligence technology (including machine learning models and deep learning models) to generate a piece of music from the rhythm of the user's movement gait.

### SUMMARY OF THE INVENTION

Based on above, one aspect of the present invention is to provide an apparatus for generating music based on gait. The apparatus comprises a sensing assembly provided on a user's foot, the sensing assembly includes a pressure sensor and an inertial sensor configured to collect data related to the user's movement. A computing device is coupled to the sensing device, wherein the computing device includes a gait analysis device and a rhythm parameter extraction module, wherein the gait analysis device is employed to analyze a movement data to obtain gait parameters, and the rhythm parameter extraction module extracting movement rhythm parameters from the gait parameters; an AI music generation module is coupled to the computing device, and is configured to receive the movement rhythm parameters and convert the movement rhythm parameters into corresponding beats, high and low sounds, light and heavy tones, and followed by composing melody with the beats, the high and low sounds, the light and heavy tones, thereby generating a music and outputting to the computing device.

A music gait interaction module is configurated on the computing device to generate dynamic patterns in conjunction with rhythms of the music generated by the AI music generation module for interacting with the user. The AI music generation module and the music gait interaction module are computer software or instructions which are executable by the computer.

In one embodiment, the pressure sensor includes a plurality of pressure sensors to respectively detect pressure distributions of a toe area and a heel area of the foot. The inertial sensor includes an accelerometer and a gyroscope to sense the user's foot movements. wherein the inertial sensor further includes a GPS sensor.

In another one embodiment, the sensor assembly includes a microprocessor to collect and analyze signals detected by the pressure sensor, the inertial sensor, the GPS sensor, and to convert the signals into corresponding foot pressure distributions, an acceleration information and a GPS information; a memory coupled to the microprocessor to store the foot pressure distributions, the acceleration information and the GPS information; a wireless transceiver coupled to the microprocessor for wirelessly transmitting the foot pressure distributions, the acceleration information and the GPS information to an external device.

In one embodiment, the gait parameters include number of kicks, stride width, step length, cadence, standing time, forward, backward, jumping and movement trajectories of the user.

In one embodiment, the movement rhythm parameters include rhythm, speed, jump height, strength, stride length, and foot rotation range and frequency.

In one aspect, the AI music generation module is set up in a cloud server or the computing device. The cloud server is a generative AI server. In one aspect, the wireless transceiver includes a Bluetooth or WiFi device.

According to another aspect of the present invention, the present invention discloses a method of generating music based on gait. The present invention provides a pressure sensor and an inertial sensor configured to collect data related to the user's movement. Then, a gait analysis device is employed to analyze a movement data to obtain gait parameters, and a rhythm parameter extraction module is employed to extract movement rhythm parameters; an AI music generation module is configured to receive the movement rhythm parameters and convert the movement rhythm parameters into corresponding beats, high and low sounds, light and heavy tones, and followed by composing melody with the beats, the high and low sounds, the light and heavy tones, thereby generating a music and outputting to the computing device.

In the present disclosed method, the gait parameters include number of kicks, stride width, step length, cadence, standing time, forward, backward, jumping and movement trajectories of the user. The movement rhythm parameters include rhythm, speed, jump height, strength, stride length, and foot rotation range and frequency.

### BRIEF DESCRIPTION OF THE DRAWINGS

Figure 1 shows the system of the gait-based AI-generated music apparatus of the present invention.
Figure 2 shows the schematic diagram of sensors and circuits according to the present invention.
Figure 3 shows the functional block diagram of the sensors according to the embodiment of the present invention.
Figure 4 shows the diagram of the sensing assembly and the external computing device according to the present invention.
Figure 5 shows the functional block diagram corresponding to Figure 1.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The present invention disclosed an apparatus to generate AI music based on gaits. The present invention employs AI algorithms to generate music based on information related to the user's movement and gait. For example, in a badminton match, contestants have many gait-related parameters (or gait parameters) such as jumping, advancing, retreating, and so on. The rhythm, speed, jump height, strength, stride can be extracted from these gait-related parameters. These parameters are corresponding to the beats, high sounds, low sounds, light and heavy sounds, these data may be used to compose a music. The subsequent step is to compose the music by adding melody into the information using the AI algorithm to create the AI music related to movement.

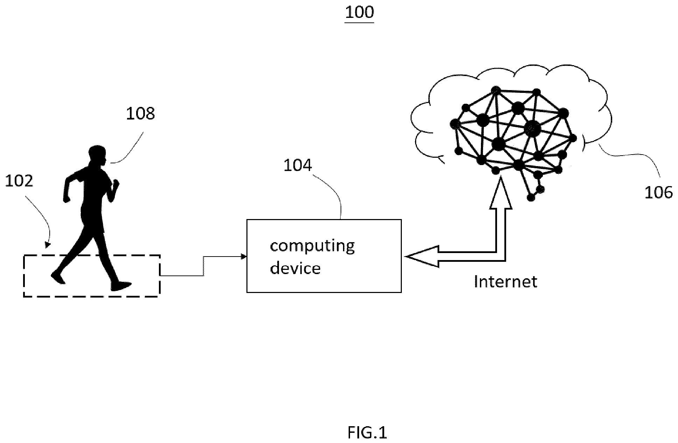

Based on the above, please refer to FIG. 1, which shows a system 100 for implementing the aforementioned gait-based AI-generated music. The system 100 includes a sensor assembly 102, a computing device 104 and a cloud server 106. The sensor assembly 102 is communicatively connected to the external computing device 104, and the external computing device 104 receives data collected by the sensor assembly 102. The computing device 104 communicatively connects to the cloud server 106 through the Internet. According to embodiments of the present invention, the cloud server 106 may be a general network server or a generative AI server.

According to an embodiment of the present invention, the sensor assembly 102 has a wireless transceiver configured to wirelessly transmit data related to the movement of the user 108 collected by the sensor assembly 102 to the receiver of the external computing device 104. As discussed in greater detail below, the sensor assembly 102 may be worn on the user 108, such as in the shoe or on the foot (feet) of the user 108, and the sensor assembly 102 includes a plurality of pressure sensor and inertial sensor module, and transceiver. According to one embodiment of the present invention, the pressure sensors and/or wireless transceivers may be separated or integrated together. According to embodiments of the present invention, the sensor assembly 102 can be worn on both feet of the user. The signals (including the data related to the movement of the user 108) generated by the sensor assembly 102 are transmitted to the computing device 104 by the wireless transceiver (not shown), the computing device 104 includes, for example, a smartphone, laptop, tablet, or personal computer.

According to an embodiment of the present invention, the aforementioned gait data of the user 108, for example, jumping, forward, backward data are received and analyzed by the computing device 104 through wireless transmission, and followed by obtaining the gait parameters and followed by uploading the data to the cloud server 106 via the internet for subsequent analysis and music generation. The system 100 also includes an application program installed on the computing device 104 to receive and process the data from the sensor assembly 102, the computing device 104, and the cloud server 106. The application can be executed based on Android, Windows or iOS platforms, and it may also upload data to the cloud server 106 for storage and/or further computing processing. The system 100 continuously collects movement data of the user 108 through the computing device 104 which executes algorithms for gait analysis, and extracts gait parameters from the user movement data.

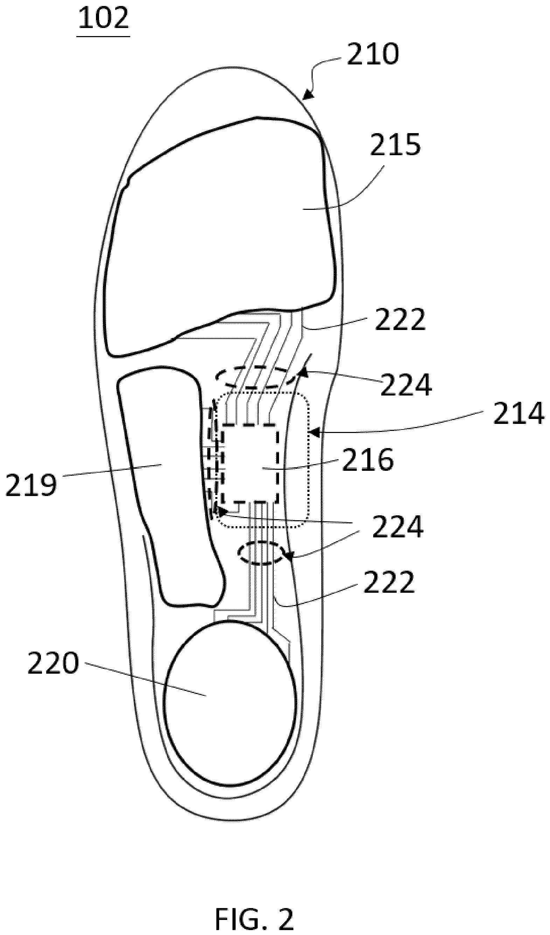

Please refer to FIG. 2, the sensor assembly 102 includes a plurality of pressure sensors and inertial sensing modules. The multiple pressure sensors and the inertial sensing modules may be integrated together or separated. For convenience of illustration, FIG. 2 only shows that the pressure sensors and inertial sensing modules are integrated in the sensor assembly 102 in the insole 210. Other sensor assembly 102 may be used without departing from the scope of the present invention. For example, the sensor assembly 102 is integrated in the shoes or shoe soles, these examples also fall within the scope of the present invention. Referring to Figure 2, the pressure sensors (not shown) can be provided in in the area 215 near toe, the area 219 aside the arch, and the heel area 220. The area 215 near toe refers to the toe area and the forefoot area. Each of the areas has multiple pressure sensors (not shown) and different pressure sensor density. Individual pressure sensors are electrically connected to the inertial sensing module 216 using wires 222. In one embodiment, the pressure sensor is a capacitive pressure sensor, different number of capacitive pressure sensors and corresponding wiring 224 form a flexible pressure sensing device, which is disposed on different parts of the insole 210 to sense the foot pressure distribution in different areas of the user's foot (for example, the area 215, the area 219, and the area 220). Alternatively, the pressure sensor may also be a resistive pressure sensor. The inertial sensing module 216 includes wireless transceivers (such as Bluetooth chips), and other electronic components (such as inertial measurement units, GPS sensors), these elements are all formed into the arch pad of the insole 210. In one embodiment, the inertial sensing module 216 may be a sensing module integrated by a printed circuit board, the sensing module has connection terminals electrically connected to the plurality of pressure sensing devices.

In another embodiment of the present invention, the sensor assembly 102 provides user movement data/information. The inertial sensing module 216 in the sensor assembly 102 may include (1) a wearable wireless real-time motion sensing device or an IMU (inertial measurement unit), (2) a wearable wireless instant multi-zone plantar pressure/six-dimensional motion capturing device.

According to the embodiment of the present invention, the inertial sensing module 216 includes at least an accelerometer, a gyroscope, a GPS sensor and other devices. The accelerometer and gyroscope are sensors based on Micro-Electro-Mechanical Systems (MEMS) technology, and both of them can be integrated into a single device with six degrees of freedom.

The sensor assembly 102 may be a multi-zone plantar pressure/six-axes motion detection device. When the user exercise, the sensor assembly 102 records the user's foot pressure (by the multiple pressure sensors) and motion with six degrees of freedom (through the inertial sensing module 216). In some embodiments, the foot pressure/six axes motion detection device has a variable recording interval, the sampling circuit obtains the pressure point data of each foot and fetches the foot pressure distribution at each sampling interval by the sensors located the area 215, area 219 and the heel area 220.

The sensor assembly 102 may include an inertial sensing module 216, which serves as a six-axes motion detection device (or called six-axes inertial measurement device), the devices detect motion changes based on Micro-Electro-Mechanical Systems (MEMS) sensors. The six-axes motion detection device determines accelerations Ax, Ay, Az in three dimensions, and the pitch, the yaw and the roll of rotational movements. For example, it generates 600 action data points per second by sampling at 100Hz by the sampling circuit. The foot motion data is captured for each sampling interval. Utilizing six-axes motion detection device with a multi-zone pressure sensing device (multiple pressure sensors disposed on the insole), the present invention allows to dynamically track the spatial and temporal gait during walking/exercise.

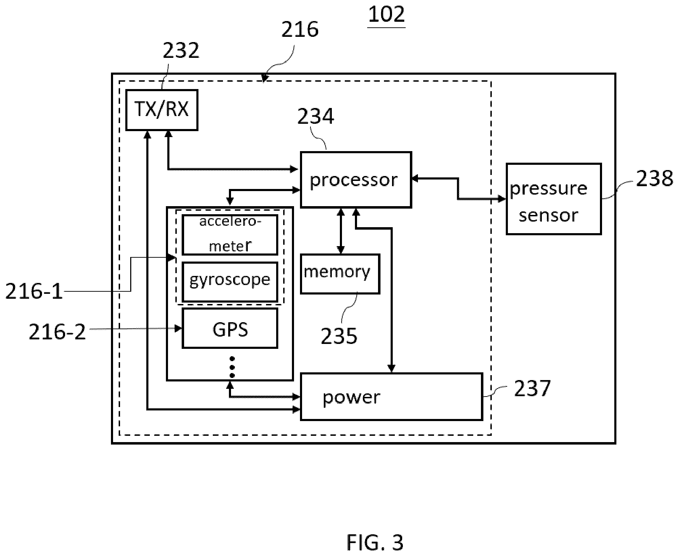

FIG. 3 shows a functional block diagram of the sensor assembly 102, which includes an inertial sensing module 216 capable of data transmission/reception through a wireless transceiver (TX/RX) 232. Although FIG. 2 shows that the wireless transceiver (TX/RX) 232 is integrated into the inertial sensing module 216, the person having ordinary skill in the art realizes that the separated wireless transceiver (TX/RX) 232 can also be used for the purpose of data transmission/reception. In the example of FIG. 3, the inertial sensing module 216 may include a wireless transceiver (TX/RX) 232 for transmitting data to and/or receiving data from one or more remote systems. In one embodiment, the low-power wireless transceiver 232 may be a Bluetooth, WiFi, RF, Zigbee, narrow band IoT (NB-IOT) networking devices, other medium/long-range wireless transceivers may also be utilized. The inertial sensing module 216 is electrically connected to the pressure sensors (pressure sensing devices 238) provided on the insole via connection terminals. The inertial sensing module 216 also includes one or more microprocessors 234, a memory 235, an integrated inertial sensor 216-1 (including an accelerometer, a gyroscope (G-sensor)), and a GPS sensor 216-2 and a power supply device 237. The power supply device 237 provides power to the pressure sensing device 238, and other devices (e.g., microprocessor 234, memory 235, composite sensor 216-1, GPS sensor 216-2 etc.) of the sensor assembly 102. In a preferred embodiment, the power supply device 237 includes a rechargeable solid-state battery, an inductive coil for coupling with an external wireless charging system to wirelessly charge the battery, and a USB charging interface. It should be note that the sensor assembly 102 provides a computer program/algorithm to collect and store user's body movement data, for example, the user's foot pressure distribution, the impact force, the user's movement trajectory (forward, backward, jump), speed, distance, acceleration, angular orientation and angular orientation change data, etc., and these programs/algorithms can be stored and/or executed.

The wireless transceiver 232 connects to at least one pressure sensors, and the inertial sensors 216-1, GPS sensors 216-2, which detect and provide various data parameters. These data and information include user physiological data including user's foot pressure distribution, impact, the movement trajectory, speed, distance, GPS, acceleration, angular orientation data, and angular orientation changes (from the gyroscope sensor) data parameters which may be stored in memory or transmitted to remote computing electronics or servers via wireless transceiver 232.

The inertial sensing module 216 may also be configured to communicate with an external computing device 104, which may include, for example, a smartphone, a laptop, a tablet, or a computer.

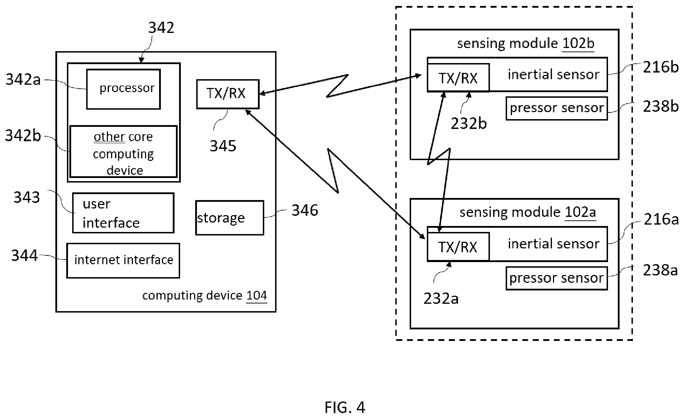

From a system perspective, as shown in Figure 4, the user 108 employs two sensor assemblies 102 for each foot (one for each foot), for example, the sensing modules (102a, 102b) are provided on the left and the right insoles respectively.

FIG. 4 shows a system block diagram of sensing modules (102a, 102b) which communicate with the external computing electronic device 104. The sensing modules (102a, 102b) are provided on the feet of the user 108. For example, each one of the left and right insoles include an inertial sensing module (216a, 216b) formed in the arch portion of the insole, and is electrically connected to the pressure sensing device (238a, 238b) to receive and analyze the user's foot pressure distribution data, and transmit the above data to the wireless transceiver (232a, 232b) located in the sensing module, the remote computing device or server. Each one of the inertial sensing modules (216a, 216b) include a processing device (one or more microprocessors), memory, additional sensors, and a power supply device (refer to FIG. 2).

The external computing device 104 could be any electronic device that can transmit, process, and store data. In one embodiment, external computing device 104 is a portable computing device including a social networking device, a gaming device, a cell phone, a smartphone, a personal digital assistant, a digital audio/video player, a laptop, a tablet, a video game controller, or any other portable device having computing core.

The external computing device 104 includes a computing core 342, a user interface 343, an internet interface 344, a wireless communication transceiver 345, and a storage device 346. The user interface 343 includes one or more input devices (for example, keyboard, touch screen, voice input device, etc.), one or more audio output devices (for example, speakers, headphone jack, etc.), and/or one or more visual output devices (such as video graphics displays, touch screens, etc.). The internet interface 344 includes one or more networking devices (for example, wireless local area network (WLAN) devices, wired LAN devices, wireless wide area network (WWAN) devices, etc.). The storage 346 includes flash memory, one or more hard drives, one or more solid state (SS) storage devices, and/or cloud storage.

The computing core 342 includes a processor 342a and other computing core components 342b which include one or more video graphics processing unit, memory controllers, main memory (RAM), input/output (I/O) device interface modules, input/output (I/O) interfaces, input/output (I/O) controllers, peripheral device interfaces, USB interface modules, network interface modules, memory interface modules and peripheral device interface module.

The wireless communication transceiver 345 of the computing device 104 and the wireless transceivers (232a, 232b) of the sensing modules (102a, 102b) are the similar transceiver types, for example, Bluetooth, WLAN, Wifi, etc., the wireless transceivers (232a, 232b) communicate directly with the wireless communication transceiver 345 to share collected data through their respective sensing modules (102a, 102b) and/or receive instructions from external computing devices 104. Alternatively, the wireless transceivers (232a, 232b) communicate with one another for collecting data. The wireless transceivers (232a, 232b) transmit the collect data to the wireless communication transceiver 345 of the external computing device 104.

According to an embodiment of the present invention, referring to Figures 1-4, when the user 108 steps on the pressure sensor, the pressure sensing device (238a, 238b) is activated. When the user moves, jumps or runs, the inertial measurement module (216a, 216b) is activated as well. Both the pressure sensing devices (238a, 238b) and the inertial measurement modules (216a, 216b) send electrical signals to the external computing device 104. The microprocessor 234 of the sensor assembly 102 can be used to detect and analyze user's feet data during exercise, user's feet data includes, for example, the pressure wave curve (the curve of pressure changing with time), acceleration curve (the curve of Ax, Ay, Az changing with time) or angular curve (the curve of angular velocity (ωx, ωy, ωz) changes with time), followed by converting these continuous pressure waves and acceleration curves into discrete pulse signals.

According to an embodiment of the present invention, the gyroscope in the inertial measurement module (216a, 216b) can sequentially integrate the acceleration Ax, Ay and Az along the X, Y and Z axes to obtain velocities along axes Vx, Vy and Vz. Similarly, the gyroscopes sequentially integrate the angular velocities ωx, ωy and ωz rotating around the X, Y and Z axes to obtain the pitch angle (θ), roll angle (γ) and yaw angle (ψ).

During typical body movements, such as walking, dancing, or ball games, people alternate their left and right feet during walking. In order to generate a musical scale from these alternating footsteps, each signal pulse sent from the wireless transceiver (232a, 232b) of the sensing module (102a, 102b) is sequentially numbered by the processor.

The present invention analyzes the user's gait-related information by the pressure wave curve (pressure changes with time) detected by the pressure sensing device (238a, 238b). For example, the length of stride time of the user 108 can be measured by the time difference between consecutive heel strikes of the same foot of the user 108, while the user's swing time (i.e., the length of time the foot swings in the air) can be measured by the time difference between toe off and heel strike of the same foot. The standing time (i.e., the length of time the foot is in contact with the floor) of the user 108 can be measured by the time difference between heel strike and toe off. These data may also be cooperated with the data received by the inertial sensing module (216a, 216b) measurement units and the GPS sensor 236 to enable measurement of step length or distance and other gait characteristics.

According to the embodiment of the present invention, the heel strike pressure signal corresponds to the foot pressure distribution signal sensed by the pressure sensor in the heel area 220 in Figure 2, and the pressure signal of the toe off the ground (toe off) corresponds to the foot pressure signal sensed by the pressure sensor disposed near the toe area 215 in FIG. 2.

According to aforementioned, the information, for example, the number of kicks, stride length, step length, step frequency, and standing time can be obtained by analyzing the pressure wave curve (pressure changes with time) of the user's 108 feet. Further information such as user's forward, backward, jumping, motion trajectory is obtained by the accelerometer, GPS sensor of the inertial sensing module (216a, 216b). In addition, the rotation movement is detected by the gyroscope (216a, 216b).

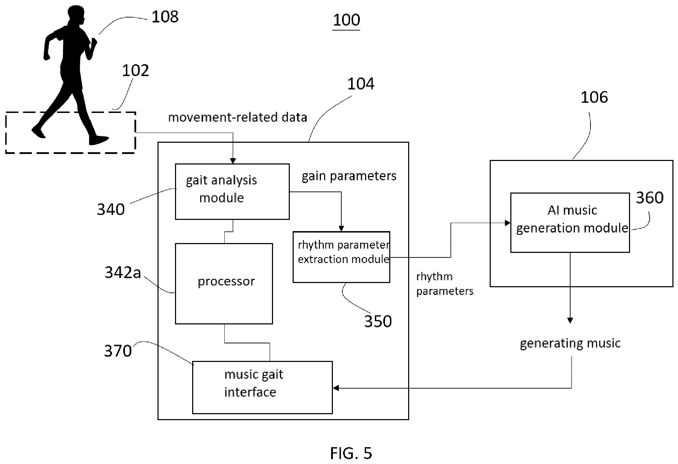

Referring to Figure 5, it shows a functional block diagram corresponding to the system of Figure 1, the external computing electronic device 104 receives the motion data of the user 108, that is, the time dependent pulse signals of pressure and acceleration. The data is processed by the gait analysis module 340 to generate various gait parameters. For example, the processor 342 of the external computing electronic device 104 cooperates with the computing or algorithm in the gait analysis module 340 to process the pulses of pressure, acceleration and angular velocity from the sensor modules (102a, 102b). The external computing device 104 analyzes the time dependent pressure, acceleration and angular velocity on the left and right feet of the user 108 by the gait analysis algorithm set in the gait analysis module 340 using the collected movement-related data of the user. The pulse signal, the collected GPS data are analyzed to obtain the user's 108 gait parameter information, for example, the number of kicks, stride length, step frequency, standing time, jumping, forward, backward, foot rotation and movement trajectory, followed by outputting these data. Subsequently, these output gait-related parameters are extracted by a rhythm parameter extraction module 350 to extract rhythm, speed, jump height, force, step spoke, and foot rotation range, frequency and other related motion rhythm parameters. These motion rhythm parameters can be transmitted to the AI music generation module 360 of the cloud server 106 via the internet, thereby converting the rhythm, speed, jump height, force, stride, and rotation range, frequency, etc. into related motion rhythms. The motion rhythms are then converted into the corresponding beat number, high and low notes, light and heavy notes, etc., and the AI algorithm is used to compose the melody using above data to generate a piece of music and store the generated music in the memory. Therefore, an AI music related to movement can be created. The AI music related to movement is output by the speakers.

According to an embodiment of the present invention, the user may interact with the music generated by the AI music generation module 360 by the music gait interaction interface displayed on the external computing device 104. For example, the music gait interactive module 370 is an application program (APP), which is installed into a group online game device with interesting rehabilitation functions, and the rehabilitation levels can be divided by game difficulty and scoring mechanisms. The aforementioned group online game device may include a mobile phone dancing machine application (APP), for example, the APP generates and displays dynamic patterns in accordance with the rhythm of the music generated by the AI music generation module 360 for interacting with the user for training purpose. For example, the method may train the users to perform rehabilitation according to the gait rhythm (generated by a mobile phone dance machine).

According to the embodiment of the present invention, the aforementioned AI music generation module 360 may include Amper Music, AVIA, Ecrett Music, Soundraw, Boomy, MuseNet, Amadeous2/3xo4n42k7 or other similar AI music generators, which can make music with machine learning.

According to an alternative embodiment of the present invention, the system of FIG. 5 may include multiple wireless signals generated by the physical movements of multiple individuals (multiple users), the multiple signals are generated from the feet of the multiple users. The corresponding pressure, acceleration and GPS data detected by the sensors. In one embodiment, the multiple users may be members of a dance group or competitors in a sporting event.

In the preferred embodiment, the multi-person system has a group of people, each member of which is equipped with a corresponding sensor assembly, each sensor assembly has a wireless transceiver. Each corresponding sensor and wireless transceiver may communicate with the wireless transceiver 345 of the computing device 104.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. An apparatus (100) for generating music based on gait comprising:
a sensing assembly (102) provided on a user's foot, said sensing assembly (102) including a pressure sensor (238) and an inertial sensor (216-1) configured to collect pressure, acceleration, and rotation data related to said user's movement;
a computing device (104) coupled to said sensing assembly (102), wherein said computing device (104) includes a gait analysis module (340) and a rhythm parameter extraction module (350), wherein said gait analysis module (340) is employed to analyze said pressure, acceleration, and rotation data to obtain gait parameters, and said rhythm parameter extraction module (350) extracting rhythm parameters from said gait parameters;
an AI music generation module (106) coupled to said computing device (104) to receive and convert said rhythm parameters into corresponding beats, high and low sounds, light and heavy tones, and followed by composing melody with said beats, said high and low sounds, said light and heavy tones, thereby generating a music and outputting to said computing device (104).

2. The apparatus of claim 1, further comprising a music gait interaction module (370) configurated on said computing device (104) to generate dynamic patterns with rhythms of said music generated by said AI music generation module (106) for interacting with said user.

3. The apparatus of claim 1, wherein said pressure sensor (238) includes a plurality of pressure sensors to respectively detect pressure distributions of a toe area (215) and a heel area (220).

4. The apparatus of claim 1, wherein said inertial sensor includes an accelerometer and a gyroscope to sense said user's foot movements.

5. The apparatus of claim 4, wherein said inertial sensor (216-1) includes a GPS sensor.

6. The apparatus of claim 4, wherein said sensor assembly (104) includes a processor (342a) to collect and analyze signals detected by said pressure sensor (238), said inertial sensor (216-1), a GPS sensor (216-2), and to convert said signals into corresponding foot pressure distributions, an acceleration information and a GPS information.

7. The apparatus of claim 6, sensor assembly (104) includes a memory (346) coupled to said processor (342a) to store said foot pressure distributions, said acceleration information and said GPS information.

8. The apparatus of claim 7, sensor assembly (104) includes a wireless transceiver (345) coupled to said (342a) for wirelessly transmitting said foot pressure distributions, said acceleration information and said GPS information.

9. The apparatus of claim 8, wherein said wireless transceiver (345) includes a Bluetooth or WiFi device.

10. The apparatus of claim 1, wherein said gait parameters include number of kicks, stride width, step length, cadence, standing time, forward, backward, jumping and movement trajectories of said user.

11. The apparatus of claim 1, wherein said rhythm parameters include rhythm, speed, jump height, strength, stride length, and foot rotation range and frequency.

12. The apparatus of claim 1, wherein said AI music generation module (360) is set up in a cloud server (106) or said computing device (104).

13. The apparatus of claim 12, wherein said cloud server (106) is a generative AI server.
